# EUROPEAN PATENT SPECIFICATION

(11) **EP 2 432 495 B1**
(45) Date of publication and mention of the grant of the patent: **22.03.2017**
(21) Application number: 10778380.5
(22) Date of filing: 20.05.2010
(51) Int. Cl.: A61K 38/39, A61K 31/715, A61K 47/50, A61P 17/00, A61P 29/00

(54) **ELASTIN FOR SOFT TISSUE AUGMENTATION**
ELASTIN ZUR WEICHGEWEBEVERGRÖSSERUNG
ELASTINE POUR L'AUGMENTATION DES TISSUS MOUS

(30) Priority: 20.05.2009 US 179875 P
(43) Date of publication of application: 28.03.2012
(73) Proprietor: Humacyte, Inc., Research Triangle Park, NC 27709 (US)
(72) Inventor: NIKLASON, Laura, E., Greenwich CT 06831 (US); LI, Yuling, Chapel Hill NC 27514 (US); PRICHARD, Heather, Raleigh NC 27615 (US); DAHL, Shannon, Durham NC 27713 (US); BLUM, Juliana, Raleigh NC 27613 (US)
(74) Representative: Cooley (UK) LLP
(86) International application number: PCT/US2010/035568
(87) International publication number: WO 2010/135527

(56) References cited:
- US-A1- 2004 078 090
- US-A1- 2005 058 629
- US-A1- 2006 093 644
- US-A1- 2007 071 729
- US-A1- 2008 038 306
- US-A1- 2009 028 817
- DATABASE WPI Week 199412 Thomson Scientific, London, GB; AN 1994-097757 XP002689917, & JP 6 048915 A (TSUJI T) 22 February 1994 (1994-02-22)
- DAAMEN WILLEKE F ET AL: "Isolation of intact elastin fibers devoid of microfibrils", TISSUE ENGINEERING, vol. 11, no. 7-8, July 2005 (2005-07), pages 1168-1176, XP002689912, ISSN: 1076-3279

## Description

This invention relates generally to compositions comprising elastin and kits for use in therapeutic and non-therapeutic methods for soft tissue augmentation.

### BACKGROUND OF THE INVENTION

Natural skin is composed of many elements, including dermal fibroblasts and keratinocytes, hair follicles, nerves and blood vessels. Extracellular matrix components of skin, which are responsible for the strength, elasticity and turgor of native, healthy skin, include collagens, elastin and glycosaminoglycans. Collagen molecules provide the bulk of the tensile properties of all connective tissues in the human body, including skin. Elastin is a very long-lived protein that nonetheless breaks down in the skin of older individuals. Elastin breakdown contributes to skin drooping and wrinkles. Hydration is retained in skin by the presence of glycosaminoglycans, which act as "sponges" to retain water and provide skin with its natural turgor. Without these critical extracellular matrix components, skin becomes thin, wrinkled, and weak.

Various forms of injectable products have been developed for skin and other soft tissue augmentation. These products fall into synthetic and "natural" categories, wherein natural materials are derived from animal or human tissues. Synthetic materials that have been used as tissue bulking agents include silicone, oils and waxes, but these materials suffer from healing complications and are very viscous and difficult to inject. Animal-derived materials that have been described include bovine collagen in injectable forms. However, bovine collagen induces occasional immune reactions in recipients, due to the fact that bovine collagens are not identical to human collagens and can serve as antigens for immune reactivity. Other animal-derived extracellular matrix materials include hyaluronic acid that is derived from rooster combs. This material is quite viscous and also has the drawback of being of non-human origin. Additionally, various preparations of elastin currently in use have the drawback of inducing calcification upon implantation.

Substances that are injected into the skin are often perceived by the host as foreign bodies. Typical adverse host responses to substances injected into the skin include, but are not limited to, inflammation, recruitment of fibroblasts and fibrous encapsulation, recruitment of leukocytes and foreign body giant cells, calcification, scarring, and immune response with generation of antibodies or activated T-cell responses. All synthetic biomaterials, such as silicone, polylactic acid, Teflon and other polymers, metals and plastics, elicit some degree of foreign body reaction when implanted into the skin. Such adverse responses to foreign implanted materials lead to many of the undesirable effects of dermal filler products, including but not limited to, lumpiness, fibrous encapsulation, scarring, calcification, migration of implanted materials, breakdown of the implanted materials that leads to lack of persistence, and chronic inflammation and irritation as well as potential immune response. An ideal dermal filler material would not elicit such adverse responses in the host skin tissue. In addition, an ideal dermal filler would remain inert and non-inflammatory over long periods of time, i.e. months.

In general, extracellular matrix proteins elicit less inflammatory, calcification and fibrous encapsulation response than synthetic materials and polymers. However, if extracellular matrix proteins are derived from allogeneic sources as opposed to autologous sources, then the adverse host responses are typically increased as compared to autologous sources of materials. In addition, if extracellular matrix proteins are derived from xenogeneic as opposed to autologous or allogeneic sources, then adverse host responses are typically further increased after implantation into the skin or sub-dermal space.

The compositions for use or as used in the present invention address these problems in the art with many dermal fillers, and fulfill a long felt need in the art of dermal and soft tissue filling.

### SUMMARY OF THE INVENTION

The present invention provides a composition for use in a therapeutic method for soft tissue augmentation in a human subject, wherein said composition comprises isolated non-human elastin and a pharmaceutically acceptable carrier, wherein the elastin has a molecular weight greater than 100 kDa and is substantially insoluble in water, and wherein the non-human elastin is isolated from non-frozen vascular tissue. The invention also provides use of a composition in a non-therapeutic method for soft tissue augmentation in a human subject, wherein said composition comprises isolated non-human elastin and a pharmaceutically acceptable carrier, wherein the elastin has a molecular weight greater than 100 kDa and is substantially insoluble in water, and wherein non-human the elastin is isolated from non-frozen vascular tissue.

The present disclosure also provides a composition comprising isolated non-human elastin and a pharmaceutically acceptable carrier wherein the elastin has a molecular weight greater than 100kDa and is substantially insoluble in water.

The present disclosure additionally provides a composition comprising isolated human elastin and a pharmaceutically acceptable carrier wherein the elastin has a molecular weight greater than 100 kDa and is substantially insoluble in water, and wherein the composition does not comprise human collagen.

The compositions can include isolated human elastin derived from engineered vascular tissue or native vascular tissue. The compositions can include elastin that is non-human in origin, or that is isolated from tissues that are non-vascular, such as skin, tendon, ligament, lung or placenta. The isolated elastin can be cross-linked or not. The elastin in the composition can be autologous, allogeneic, or xenogeneic to the subject or recipient. Preferably, the subject is a mammal. More preferably, the subject is a human.

The compositions can include about 2 to about 1000 mg/ml of isolated elastin, preferably about 10 to 60 mg/ml of isolated elastin. The compositions can be formulated for parenteral administration. Preferably, the compositions are provided in an injectable form.

The elastin can be suspended in any pharmaceutically acceptable carrier, or may be delivered in a dehydrated form. Such acceptable carriers might include, but are not limited to, saline, physiological buffer solution, collagen that is autologous, allogeneic or xenogeneic to the recipient, glycosaminoglycans such as hyaluronic acid, glyerine, carboxymethylcellulose, polyethyleneglycol, or other biocompatible materials, or any combination of such biocompatible materials. The collagen may be isolated. The collagen may be of human or non-human origin.

The elastin may be fixed or cross-linked in order to increase acceptance by the host, decrease immunogenicity, or improve persistence of the injected material in the host. The material in which the elastin is suspended may be fixed, cross-linked, or otherwise chemically altered so as to increase acceptance by the host, decrease immunogenicity, or improve persistence of the injected material. Examples of fixation, chemical cross-linking or other chemical treatments include, but are not limited to, fixation in alcohols, fixation in aldehydes such as glutaraldehyde, cross-linking by glutaraldehyde or by reducing sugars, enzymatic treatment to remove portions of molecules such as telopeptide fragments of collagen, cross-linking by photo-initiated reactions, and other chemical treatments. The material in which the elastin is suspended may be treated by physical means in order to improve purity, achieve a desired particle size or solution viscosity, or alter other physical characteristics.

The compositions can further include one or more active agents selected from the group consisting of one or more anti-inflammatory agents, tissue formation agents, adipose tissue formation agents, anesthetics, antioxidants, heparin, epidermal growth factor, transforming growth factor, transforming growth factor-β, platelet-derived growth factor, fibroblast growth factor, connective tissue activating peptides, β-thromboglobulin, insulin-like growth factors, tumor necrosis factors, interleukins, colony stimulating factors, erythropoietin, nerve growth factors, interferons or combinations thereof. The compositions can further comprise one or more cells or tissues, preferably adipose tissue or dermal fibroblasts. The compositions can also include drugs for localized delivery.

The compositions can further include elastin isolated from non-frozen vascular tissue. Alternatively, elastin can be isolated from tissues that have been previously frozen, vitrified, or otherwise cryopreserved.

The compositions do not induce calcification *in vivo* upon administration to a recipient or subject. The compositions also do not stimulate host fibrotic or induce long-term inflammatory response or encapsulation *in vivo* upon administration to a recipient or subject.

The present disclosure also provides methods for soft tissue augmentation in a subject in need thereof comprising, administering the compositions of the present invention. The method of the soft tissue augmentation can improve conditions including, but not limited to, lines, folds, wrinkles, minor facial depressions, cleft lips, correction of minor deformities due to aging or disease, deformities of the vocal cords or glottis, deformities of the lip, crow's feet and the orbital groove around the eye, breast deformities, chin deformities, augmentation; cheek and/or nose deformities, acne, surgical scars, scars due to radiation damage or trauma scars, and rhytids. The method of soft tissue augmentation can increase tissue volume. The compositions may be injected into the skin or may be injected underneath the skin. The compositions may be used in the treatment of urinary incontinence, vesicoureteral reflux, anal incontinence, gastric reflux, or other soft tissue areas wherein bulking of tissues can exert a therapeutic effect. The compositions include insoluble elastin that does not induce encapsulation, an inflammatory, immune or fibrotic response and does not induce calcification *in vivo* upon administration.

The present invention includes dermal or subdermal fillers containing the compositions for use or as used according to the present invention. The present invention also includes kits and using the kits for augmentation of a soft tissue. The present kits include the compositions as described above, a sterile wrapper surrounding said syringe and providing a sterile environment for said syringe and one or more reagents. The reagents are selected from the group consisting of collagen, hyaluronic acid, glycerine, carboxymethyl cellulose, polyethylene glycol, lidocaine, bupivicaine, viscous biocompatible carriers, heparin, epidermal growth factor, transforming growth factor, transforming growth factor- β, platelet-derived growth factor, fibroblast growth factor, connective tissue activating peptides, β-thromboglobulin, insulin-like growth factors, tumor necrosis factors, interleukins, colony stimulating factors, erythropoietin, nerve growth factors, interferons, osteogenic factors and bone morphogenic proteins.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

Other features and advantages of the invention will be apparent from the following detailed description and claims.

### BRIEF DESCRIPTION OF THE DRAWINGS

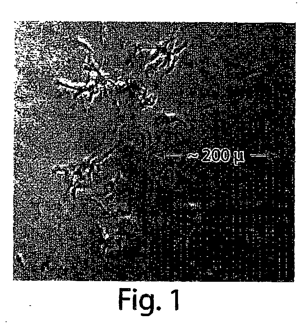

Figure 1 illustrates fibers of elastin that are obtained in accordance with the present invention, showing that fibers are generally less than 200 microns in length, though some fibers may be longer than this.

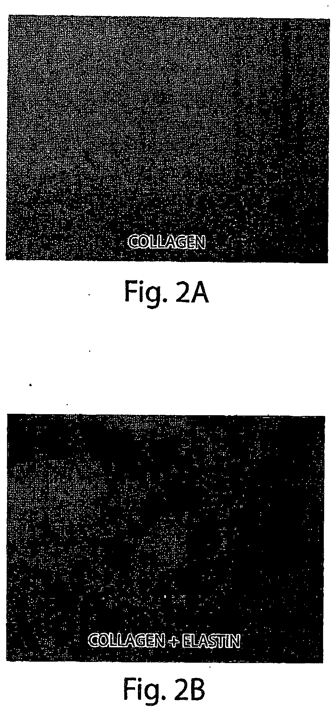

Figure 2 illustrates an example of one means of preparing an injectable formulation of elastin in accordance with the present invention. Panel (A) shows a phase-contrast micrograph of a gel suspension of porcine collagen. Panel (B) shows a phase-contrast micrograph of a porcine collagen gel, into which human elastin fibers have been suspended.

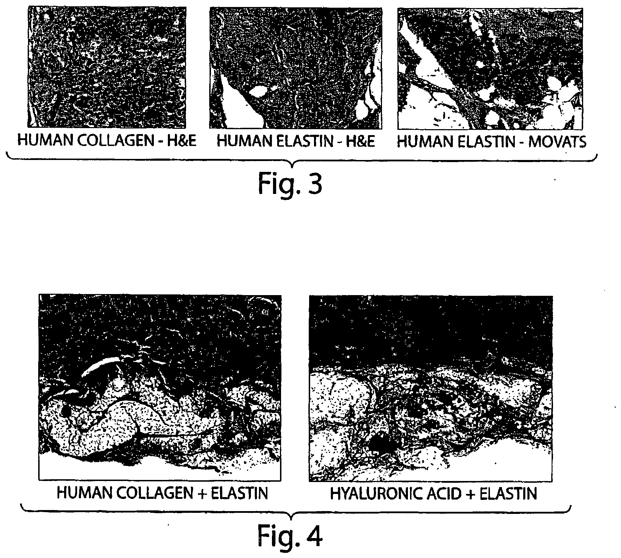

Figure 3 illustrates the histological appearance after 4 months of xenogeneic, purified extracellular matrix proteins that are injected subcutaneously into the skin of a porcine recipient. Purified human collagen shows extensive infiltration of host inflammatory cells on H&E stain, while human elastin that is prepared in accordance with the present invention shows minimal host cell infiltration and no inflammation on H&E stain. Presence of injected human elastin into the subcutaneous space is confirmed by Movats staining, which reveals dense black staining of injected elastin.
Figure 4 illustrates Movats micrographs of subcutaneous injections of two different preparations into porcine skin after 4 months. Elastin mixed with human collagen shows persistence of elastin (stains black) after 4 months, while there is little evidence of human collagen remaining. Elastin mixed with commercial cross-linked hyaluronic acid product (Restylane stains blue) shows persistence of elastin and hyaluronic acid, with little inflammatory response.

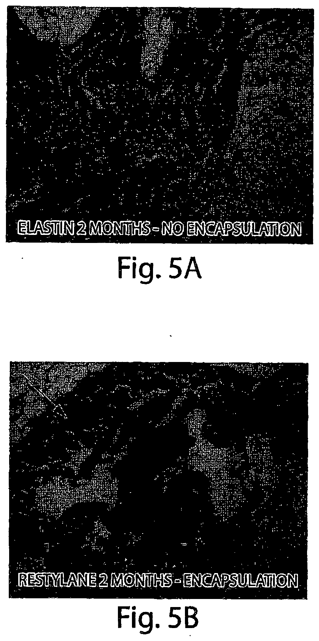

Figure 5 illustrate Movats micrographs of subcutaneous injections of two different preparations into porcine skin after 2 months. Panel (A) Injection of human elastin + collagen into pig subcutaneous space shows no encapsulation response to the implant. Panel (B) In contrast, crosslinked hyaluronic acid product (Restylane) shows substantial encapsulation by host cells and deposition of fibrous collagen after 2 months of subcutaneous implantation. This figure shows the response of the host to elastin that is xenogeneic (human into pig) to the recipient.

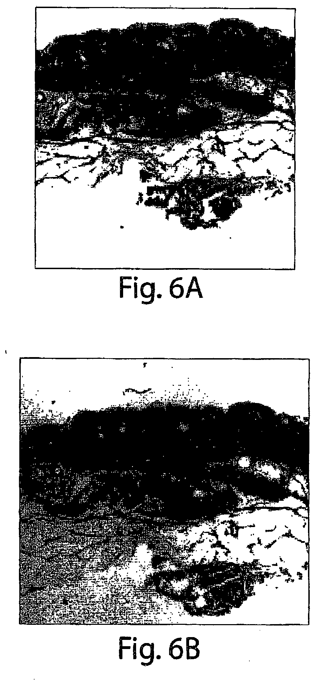

Figure 6 illustrates H&E and Movats micrographs of subcutaneous injections of porcine elastin (30 mg/mL) in porcine collagen (10 mg/mL) into a porcine recipient after 1 month. Panel (A) H&E stain; Panel (B) Movats stain, elastin stains black. Minimal host response to injected collagen that isi allogeneic to the recipient (pig elastin into allogeneic pig recipient).

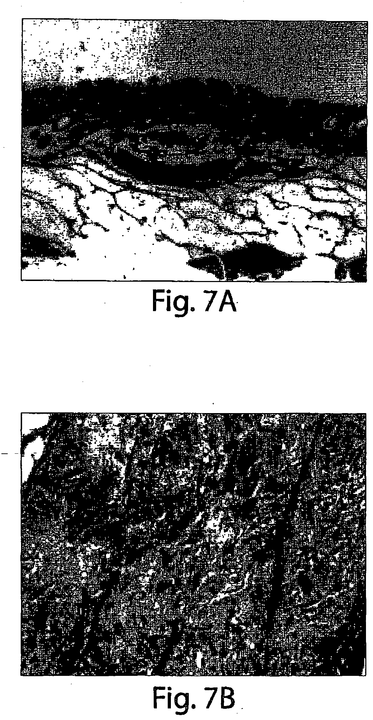

Figure 7 illustrates Movats (low power) and H&E (high power) of subcutaneous injections of human elastin (45 mg/mL) in porcine collagen (10 mg/mL) into a porcine recipient after 1 month, when host inflammatory response would be expected to be maximal. Panel (A) Movats stain at low power, elastin stains black; Panel (B) H&E stain at high power, showing few infiltrating cells into human elastin and minimal inflammatory response.

### DETAILED DESCRIPTION OF THE INVENTION

The disclosure describes the preparation of an extracellular matrix protein material, purified insoluble elastin, that elicits essentially no adverse host response when implanted into the skin or sub-dermal space. The minimal adverse host response to elastin that is prepared in accordance with the present invention is a novel and unanticipated finding. In addition to the lack of adverse host response, the elastin that is prepared in accordance with the present invention persists after injection into the skin longer than other injected extracellular matrix proteins, including collagen. The elastin also persists after injection into the skin longer than other types of injectable dermal fillers, such as cross-linked hyaluronic acid. While not subscribing to any particular theory of the invention, the long persistence of elastin after injection into the skin may be due to the lack of adverse host response that is stimulated by the material.

The elastin that is prepared in accordance with the present invention is preferably implanted by injection, but may be implanted by surgical procedures as well. The elastin that is prepared in accordance with the present invention may be obtained from tissues from any mammalian species, including but not limited to human, porcine, bovine, or primate, and may be successfully implanted without adverse host response into autologous, allogeneic, or xenogeneic recipients. The elastin that is prepared in accordance with the present invention may be obtained from any of a variety of tissues, including but not limited to blood vessel, skin, tendon, ligament, ligamentum nuchae, etc.

The elastin that is prepared in accordance with the present invention is highly pure and is prepared and isolated in such a way as to provide a surprising degree of resistance to adverse host response. Other reports in the literature point to adverse host reactions in response to xenogeneic elastin (Biocompatibility of a xenogenic elastin-based biomaterial in a murine implantation model: the role of aluminum chloride pretreatment. Hinds MT, Courtman DW, Goodell T, Kwong M, Brant-Zawadzki H, Burke A, Fox BA, Gregory KW. J Biomed Mater Res A. 2004 Apr 1;69(1):55-64.). The elastin material elicits minimal or no adverse host response in terms of fibroblasts and fibrous encapsulation, recruitment of leukocytes and foreign body giant cells, calcification, or scarring. It is anticipated that this material also elicits no adverse host immune response, in terms of antibody production or T-cell activation. This minimal adverse host response is observed even when the elastin is implanted into either an xenogeneic or allogeneic recipient (eg. human elastin into porcine recipient, or porcine elastin into porcine recipient), which is a surprising finding and which contrasts strongly with purified human collagen, which elicits strong host inflammatory responses in xenogeneic porcine recipients.

In addition, the elastin that is prepared according to the present invention may be suspended and delivered in any of a range of pharmaceutically acceptable carriers or injectable substances, including but not limited to, buffers, saline solutions, collagen, glycerine, or hyaluronic acid. The elastin that is prepared according to the present invention does not elicit adverse host response when suspended in different media, including collagen and hyaluronic acid, thereby enhancing the utility of the current invention. In addition, the elastin that is prepared in accordance with the present invention is easily injected and is well tolerated by recipients.

The present invention provides compositions for use or as used in the augmentation of skin and other soft tissues. Preferably, the compositions are formulated for injection. Unlike other injectable formulations for skin augmentation that contain only collagens or only animal-derived hyaluronans, these formulations contain other extracellular matrix components, specifically elastin, that render them more similar to native, healthy human skin.

Augmentation of soft tissue, such as skin, can be an important factor in recovering from injury or for cosmetic purposes. For example, with normal aging, skin may become loose or creases can form, such as nasal-labial folds. In the face, creases or lines may adversely affect a person's self esteem or even a career. Thus, there has been a need for compositions and methods that can diminish the appearance of creases or lines.

Further, there are situations in which loss of tissue can leave an indentation in the skin. For example surgical removal of a dermal cyst, lipoatrophy or solid tumor can result in loss of tissue volume. In other cases, injuries, such as gunshot wounds, knife wounds, or other excavating injures may leave an indentation in the skin. Regardless of the cause, it can be desirable to provide a dermal filler that can increase the volume of tissue to provide a smoother or more even appearance.

One example for needed support is dermal augmentation in the face where dermal and subdermal volume is lost due to aging.

The term "soft tissue augmentation" includes, but is not limited to, the following: dermal tissue augmentation; filling of lines, folds, wrinkles, minor facial depressions, cleft lips and the like, especially in the face and neck; correction of minor deformities due to aging or disease, including in the hands and feet, fingers and toes; augmentation of the vocal cords or glottis to rehabilitate speech; hemostatic agent, dermal filling of sleep lines and expression lines; replacement of dermal and subcutaneous tissue lost due to aging; lip augmentation; filling of crow's feet and the orbital groove around the eye; breast augmentation; chin augmentation; augmentation of the cheek and/or nose; bulking agent for periurethral support, filling of indentations in the soft tissue, dermal or subcutaneous, due to, e.g., overzealous liposuction or other trauma; filling of acne or traumatic scars and rhytids; filling of nasolabial lines, nasoglabellar lines and infraoral lines. Moreover, the present invention can be directed to hard tissue augmentation .The term "hard tissue" includes but is not limited to bone, cartilage and ligament.

In addition, the elastin of the present invention could be delivered as a solid sheet or other non-injectable form, for treatment of connective tissue or soft tissue defects, as a nerve guide, as a sling to support connective tissue, ligament or tendon repair or replacement, wound closure, closure for surgical incisions or trauma, dural closure after trauma or neurosurgery, plastic reconstructive applications, plastic surgical cosmetic applications, and musculoskeletal regeneration, replacement and repair. The method of using elastin in a sheet-based form or injectable form can also be used to improve conditions including, but not limited to, cardiac repair following infarct, vascular repair for aneurysm or fistula closure or surgically damaged vessels, bladder reconstruction, rotator cuff repair, hernia repair, or ureter repair.

The term "augmentation" means the repair, decrease, reduction or alleviation of at least one symptom or defect attributed due to loss or absence of tissue, by providing, supplying, augmenting, or replacing such tissue with the compositions of the present invention. The compositions of the present invention can also be used to prevent at least one symptom or defect.

Dermal fillers are used to fill scars, depressions and wrinkles. Dermal filler substances have various responses in the dermis from phagocytosis to foreign body reactions depending on the material (Lemperle et al., Aesthetic Plast. Surg. 27(5):354-366; discussion 367 (2003)). One goal of dermal fillers it to temporarily augment the dermis to correct the surface contour of the skin without producing an unacceptable inflammatory reaction, hypersensitivity reaction or foreign body reaction that causes pain, redness or excessive scar formation for a period of time.

The ideal material for human skin augmentation would include one or more of the critical extracellular matrix elements that provide skin its mechanical properties. These elements include collagen, elastin and glycosaminoglycans. The ideal material also would not elicit an unfavorable response in the recipient after injection. Such unfavorable responses include calcification, fibrous encapsulation, immune rejection, or prolonged inflammatory response. Many types of dermal filling procedures can benefit from the use of the compositions of the present invention. The uses of the present invention are designed (but not limited) to be used to provide increased volume of a tissue that, through disease, injury or congenital property, is less than desired. Compositions can be made to suit a particular purpose, and have desired retention times and physical and/or chemical properties.

Exemplary uses of compositions for use or as used according to this invention can be particularly desirable to fill facial tissue (e.g., nasolabial folds), to increase the volume of the dermis in the lips, nose, around the eyes, the ears and other readily visible tissue. Additionally, the compositions can be desirably used to provide bulk to increase the volume of skin secondary to excavating injuries or surgeries. For example, the site around a dermal cyst can be filled to decrease the appearance of a dimple at the site of surgery.

Also disclosed are methods of skin augmentation by administering the extracellular matrix compositions of the invention to a subject in need thereof. Preferably, the methods improve skin wrinkles and/or increase skin volume. The subject or patient treated by the methods is a mammal.

The compositions may include additional proteins, or other biological molecules, and active agents as described in further detail herein.

With respect to calcification, this complication is known to exist for various purified forms of elastin, though the mechanism that causes the calcification remains unclear (Lee, et al., American Journal of Pathology 2006; 168: 490-498; Daamen et al., Biomaterials 2005; 26: 81-92; Bollinger et al., Calcified Tissue International 1988; 42: 231-236; Urry et al., Calcified Tissue Research 1976; 21: 57-65). Competing hypotheses for elastin calcification advanced by those skilled in the art include the intrinsic nature of elastin pentapeptides to induce calcification, the central role of metalloproteinases in inducing calcification and the central role of microfibril impurities in elastin calcification. However, the precise cause of elastin calcification *in vivo* remains unknown.

The compositions for use or as used according to the present invention include an effective amount of isolated elastin and a pharmaceutically acceptable carrier. The elastin is cross-linked and insoluble. The elastin is non-human and has a molecular weight greater than 100 kDa, as determined by any assay known in the art such as SDS PAGE analysis, gel permeation chromatography, light scattering, or microscopy. Moreover, the compositions comprise a particle size less than about 400 □m, preferably less than about 200 □m, more preferably less than about 100 □m. The compositions comprise about 2-1000 mg/ml of isolated elastin, preferably 3-60 mg/ml of isolated elastin. The isolated cross-linked elastin is substantially insoluble in water, wherein the water-soluble elastin content is in the range of 0.1-10 wt%, preferably in the range of 0.1-8 wt%, more preferably in range of 0.1-6 wt%,
more preferably in the range of 0.1-4 wt%, more preferably in the range of 0.1-2 wt% and most preferably in the range of 0.1-1 wt%. Alternatively, the elastin is completely insoluble in water. In some embodiments, it is preferable to have elastin with amino acid length which permits the persistence of the protein in vivo.

The purity of elastin is typically assessed by the profile of amino acids in the final product, and by the presence of desmosine cross-links, which are specific for cross-linked and insoluble elastin. The amino acid compositions of elastin from various species have been reported (Starcher et al., Analytical Biochemistry 1976; 74: 441-447). In particular, it is known that alanine residue concentrations of greater than 200/1000 total residues, and valine residues of greater than 70/1000 total residues, are consistent with highly pure elastin (Daamen et al. , Biomaterials 2001; 22: 1997-2005). However, many methods are reported for the isolation of purified elastin, and no consensus has been reached regarding the optimal method for elastin isolation and implantation (Daamen, W.F., Hafmans, T., Veerkamp, J.H., van Kuppevelt, T.H., "Isolation of intact elastin fibers devoid of microfibrils", Tissue Engineering 2005; 11: 1168-1176).

In addition to the methods described above, elastin may also be isolated from native blood vessels, skin, tendon, ligament, ligamentum nuchae, lung, or other elastic connective tissues, by means that ensure very high purity, and thus minimize chances for immune reaction, inflammation, and calcification.

An immune and inflammatory response can be measured by various assays known in the art such as, but not limited to, MHC-peptide tetramer, ELISPOT, intracellular cytokine assay. In general, a 10-50% increase in T-lymphocytes over the base line level (e.g., wild type normal state), preferably a 50% increase in T-lymphocytes, more preferably a 40% increase in T-lymphocytes, and most preferably a 30% increase in T-lymophocyte production indicates a significant immune response.

Calcification levels can be measured by various assays known in the art such as, but not limited to, atomic spectroscopy and H&E and alizarin red staining. In general, 75-99% reduction in calcification, preferably 80% reduction in calcification, more preferably a 90% reduction in calcification, most preferably 95% reduction in calcification, indicates significant reduction in calcification with the compositions of the present invention as compared to the vehicle control. That is, the compositions of the present invention do not induce significant calcification, e.g., calcification greater than 25%, preferably calcification between 5-20%, more preferably between 10 and 15% as compared to the vehicle control (or the wild type normal state in a subject prior to administration). Alternatively, calcification levels of elastin preparation are indistinguishable form vehicle control.

The compositions for use or as used according to the present invention may also include an effective amount of one or more types of collagen. Collagen may be autologous, allogeneic, or xenogeneic to the intended recipient. The collagen may be derived from native tissues such as skin, blood vessel, tendon, ligament, intestine, bone, cartilage, bladder, placenta, or other connective tissues. The collagen may be in the form of particles, fibers, or a gel. Collagens may also be isolated from engineered tissues. Collagens may be isolated by enzymatic
digestion, digestions with strong acid or base, high ionic strength salts, mechanical disruptions such as grinding, cutting or sieving, or combinations of these approaches. The collagens may be processed so as to remove the telopeptide fragments of the molecules. The collagens may be crosslinked, using any one of a variety of means known in the art.

The compositions for use or as used according to the present invention may also include an effective amount of one or more isolated glycosaminoglycans and a pharmaceutically acceptable carrier. Glycosaminoglycans may be autologous, allogeneic or xenogeneic to the recipient. Glycosaminoglycans may be isolated from native tissues such as skin, blood vessel, cartilage, rooster combs, or other tissues containing high amounts of these molecules. Glycosaminoglycans may be derived using genetic engineering technologies, and expressed in bacteria or mammalian cells and then purified from cell culture.

Glycosaminoglycans may also be isolated from engineered tissues. Engineered tissues, grown in serum-containing medium, produce an extracellular matrix with a higher content of glycosaminoglycans than corresponding native tissues. Thus, extracellular matrix synthesized during culture contains high quantities of glycosaminoglycans and is consequently more "watery" than native tissues. Hence, engineered tissues are ideal for the production and isolation of glycosaminoglycans, which bind water and confer tissue turgor to connective tissues.

To produce isolated human glycosaminoglycans, human vascular cells are cultured in medium containing high serum (i.e. > 10% by volume of serum), and after several weeks, tissues are removed from culture and treated with hyaluronidase or other glycosaminoglycan-cleaving enzymes. Supernatant from this digestion is collected, containing high molecular weight glycosaminoglycans that may be isolated using dialysis, centrifugation, or other techniques known in the art. These glycosaminoglycans may then be used to confer tissue turgor to a treated area.

In addition to the methods described above, glycosaminoglycans and hyaluronic acid may be derived from native vascular tissues. Native blood vessels are treated with a protease such as pepsin or collagenase, in order to break up confining, fibrillar extracellular matrix. In one embodiment, the native blood vessels are extracted from discarded human umbilical cords. Such protease pre-treatment exposes glycosaminoglycans and hyaluronans to aqueous solution and allows swelling. Glycosaminoglycans and hyaluronans may then be collected from vascular tissues by any of a variety of techniques known in the art, including treatment with hyaluronidase, detergent treatment, or treatment with other enzymes that cleave glycosaminoglycan moieties.

The supernatant collected from this treatment can then be purified for high molecular weight glycosaminoglycans by any of a variety of methods, including dialysis, centrifugation, immune isolation and precipitation, etc. Preferably, the glycosaminoglycans have a MW greater than 100,000 kDa.

The compositions for use or as used according to the present invention may also include an effective amount of one or more active agents and a pharmaceutically acceptable carrier. In some embodiments, it may be useful to include one or more anti-inflammatory agents, tissue formation agents, anesthetics, antioxidants and the like, cancer-treating drugs, other drugs for localized dlivery, or combinations thereof.

Anti-inflammatory agents can include, but are not limited to, naproxen, sulindac, tolmetin, ketorolac, celecoxib, ibuprofen, diclofenac, acetylsalicylic acid, nabumetone, etodolac, indomethacin, piroxicam, cox-2 inhibitors, ketoprofen, antiplatelet medications, salsalate, valdecoxib, oxaprozin, diflunisal, flurbiprofen, corticosteroids, MMP inhibitors and leukotriene modifiers or combinations thereof.

Agents that increase formation of new tissues at the site of application can include, but are not limited to, fibroblast growth factor (FGF), transforming growth factor-beta (TGF-β), platelet-derived growth factor (PDGF) and/or fragments of angiotensin II (A-II) or combinations thereof.

Anesthetics can include, but are not limited to, those used in caudal, epidural, inhalation, injectable, retrobulbar, and spinal applications, such as bupivacaine, lidocaine, benzocaine, cetacaine, ropivacaine, and tetracaine, or combinations thereof.

Antioxidants can include, but are not limited to, Vitamin C, Vitamin A, Vitamin E, P -carotene, superoxide dismutase, catalase, selenoenzyme glutathione peroxidase, ubiquinones/ubiquinols, thioredoxin reductase, propyl, octyl and dodecyl esters of gallic acid, butylated hydroxyanisole (BHA), butylated hydroxytoluene (BHT) and nordihydroguaiaretic acid or combinations thereof.

Compositions used in the invention may additionally include one or more biologically active agents to aid in the healing or regrowth of natural tissue. For example, one may incorporate factors such as heparin, connective tissue activating peptides, β-thromboglobulin, insulin-like growth factors, tumor necrosis factors, interleukins, colony stimulating factors, erythropoietin, nerve growth factors, interferons, osteogenic factors including bone morphogenic proteins, and the like.

Any drug or other agent which is compatible with the compositions and methods of manufacture may be used with the present invention. Decisions to use such drug or agent are typically made by the attending physician based on judgments about the injury or defect being repaired.

There are numerous art recognized techniques that can be used to extract extracellular matrix components from native and engineered tissues. Specific enzymes that may be used to extract collagen and elastin matrix components include, but are not limited to, collagenase; pepsin; trypsin; elastase; matrix metalloproteinases; dispase; serine proteases; other suitable proteases; high concentrations of salts such as NaCl or other salts; alkali treatment; acid treatment; Heat (for example, autoclaving, boiling, or baking); detergents (for example, SDS or CHAPS) and/or hypotonic treatment, (for example, water) or combinations of these treatments.

There are numerous art recognized techniques that can be used to isolate and purify the extracellular matrix components that are extracted from the engineered or native vascular tissues. Such methods may include, but are not limited to, centrifugation; salt precipitation of proteins such as collagen; immunoprecipitation; antibody-mediated binding to beads followed by cleavage to isolate matrix component; isolation based upon hydrophobicity/hydrophilicity (for example, extracting hydrophobic elastin by adhesion to hydrophobic substrate such as polystyrene); dialysis (to remove low molecular weight contaminants, enzymes, salt, acid, for example); drying; altering pH of solution to induce precipitation of extracellular components; inactivation of enzymes that were used for isolation; and/or chromatographic methods (for example, polyacrylamide gel electrophoresis or high performance liquid chromatography that separate components based upon charge and molecular weight); or combinations of these treatments.

There are numerous art recognized techniques that can be used to decellularize engineered or native tissues prior to extracellular matrix isolation, in order to increase the purity of the extracted matrix, enhance its biocompatibility and persistence in vivo, and to ease the isolation of selected matrix components. In one example, aqueous hypotonic or low ionic strength solutions facilitate cell lysis in engineered and native tissues through osmotic effects. Such solutions may comprise deionized water or an aqueous hypotonic buffer (e.g., at a pH of approximately 5.5 to 8, preferably approximately 7 to 7.5). Decellularization may be accomplished using a single decellularization solution, or the construct may be incubated sequentially in two or more solutions. Another approach involves immersing the construct in alternating hypertonic and hypotonic solutions.

Preferred decellularization agents include, but are not limited to, salts, detergent/emulsification agents and enzymes such as proteases, and/or nucleases. Combinations of different classes of detergents, e.g., a nonionic detergent such as Triton X-100 (tert-octylphenylpolyoxyethylene) and an ionic detergent such as SDS (sodium dodecyl sulfate) may be employed. Preferably, one or more decellularization solutions include Triton X-100, CHAPS (3-[(3-cholamidopropyl)-dimethyl-ammonio]-1-propanesulfonate), or SDS in phosphate buffered saline (PBS). Other suitable detergents include polyoxyethylene (20) sorbitan mono-oleate and polyoxyethylene (80) sorbitan mono-oleate (Tween 20 and 80), sodium deoxycholate, and octyl-glucoside. In certain preferred embodiments, various additives such as metal ion chelators, e.g., EDTA (ethylenediaminetetraacetic acid) and/or protease inhibitors are included in the decellularization solution. Suitable protease inhibitors for use in decellularization solutions include, but are not limited to, one or more of the following: phenylmethylsulfonyl-fluoride (PMSF), aprotinin, leupeptin, and N-ethylmaleimide (NEM).

Various enzymes that degrade cellular components may be included in the decellularization solution. Such enzymes include nucleases (e.g., DNAses such as DNAse I, RNAses such as RNAse A), and phospholipases (e.g., phospholipase A or C). Certain proteases such as dispase II, trypsin, and thermolysin may be of use in decellularization. The decellularization solution preferably includes a buffer. In general, a pH between about 5.5 and 8.0, preferably between about 6.0 and 7.8, more preferably between about 7.0 and 7.5 is employed. Preferred buffers include organic buffers such as Tris (hydroxymethyl) aminomethane (TRIS), (N-[2-hydroxyethyl]piperazine-N-[2-ethanesulfonic acid] (HEPES), etc. Buffers including sodium phosphate, citrate, bicarbonate, acetate, or glutamate may also be used.

Physical forces such as the formation of intracellular ice may be employed as a primary means of accomplishing decellularization or to augment the activity of decellularization solutions. One such approach referred to as vapor phase freezing involves placing the construct or tissue in an appropriate solution, e.g., a standard cryopreservation solution such as Dulbecco's Modified Eagle Medium (DMEM), 10% dimethylsulfoxide (DMSO), 10% fetal bovine serum (FBS) and cooling at a slow rate, e.g., 1-2°C. Multiple freeze-thaw cycles may be employed. Colloid-forming materials may be added to the solution to reduce extracellular ice formation while allowing formation of intracellular ice. Appropriate materials include polyvinylpyrrolidone (10% w/v) and dialyzed hydroxyethyl starch (10% w/v).

The compounds of the present invention are administered to a patient in the form of a pharmaceutical composition. A compound that is administered in a pharmaceutical composition is mixed with a pharmaceutically acceptable carrier or excipient such that a therapeutically effective amount is present in the composition.

By "pharmaceutically acceptable" is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" derivative or analog, refers to a derivative or analog having the same type of pharmacological activity as the parent compound and approximately equivalent in degree.

The terms "effective amount" or "therapeutically effective amount" refers to an amount of the compound that is nontoxic and necessary to achieve a desired endpoint or therapeutic effect (*e.g.,* act as a dermal or subdermal filler).

A variety of preparations can be used to formulate the compositions or active agents of the present invention to render the most appropriate pharmaceutical compositions. Techniques for formulation and administration may be found in "Remington: The Science and Practice of Pharmacy, Twentieth Edition," Lippincott Williams & Wilkins, Philadelphia, PA. For human administration, preparations should meet sterility, pyrogenicity, general safety and purity standards as required by the FDA. Administration of the pharmaceutical composition can be performed in a variety of ways, as described herein.

The active agent may be administered, if desired, in the form of a salt, ester, amide, prodrug, derivative, or the like, provided the salt, ester, amide, prodrug or derivative is suitable pharmacologically. Salts, esters, amides, prodrugs and other derivatives of the active agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992).

The amount of active agent (e.g. elastin, etc.) administered will depend on a number of factors and will vary from subject to subject and depend on the particular drug administered, the particular disorder or condition being treated, the severity of the symptoms, the subject's age, weight and general condition, and the judgment of the prescribing physician. The minimum amount of drug is determined by the requirement that sufficient quantities of drug must be present in a device or composition to maintain the desired rate of release over the given period of application. The maximum amount for safety purposes is determined by the requirement that the quantity of drug present cannot exceed a rate of release that reaches toxic levels. Generally, the maximum concentration is determined by the amount of agent that can be received in the carrier without producing adverse histological effects such as irritation, an unacceptably high initial pulse of agent into the body, or adverse effects on the characteristics of the delivery device such as the loss of tackiness, viscosity, or deterioration of other properties.

The term "dosage form" denotes any form of a pharmaceutical composition that contains an amount of active agent sufficient to achieve a therapeutic effect with a single administration. When the formulation is an injection, the dosage form is usually one such injection. The frequency of administration that will provide the most effective results in an efficient manner without overdosing will vary with the characteristics of the particular active agent, including both its pharmacological characteristics and its physical characteristics.

The compositions can also be formulated for controlled release or sustained release. The term "controlled release" refers to a drug-containing formulation or fraction thereof in which release of the drug is not immediate, i.e., with a "controlled release" formulation, administration does not result in immediate release of the drug into an absorption pool. The term is used interchangeably with "nonimmediate release" as defined in Remington: The Science and Practice of Pharmacy, Nineteenth Ed. (Easton, Pa.: Mack Publishing Company, 1995). In general, the term "controlled release" as used herein includes sustained release and delayed release formulations.

The term "sustained release" (synonymous with "extended release") is used in its conventional sense to refer to a drug formulation that provides for gradual release of a drug over an extended period of time, and that preferably, although not necessarily, results in substantially constant blood levels of a drug over an extended time period.

The present formulations may also include conventional additives such as opacifiers, colorants, gelling agents, thickening agents, stabilizers, surfactants, and the like. Other agents may also be added, such as antimicrobial agents, to prevent spoilage upon storage, i.e., to inhibit growth of microbes such as yeasts and molds. Suitable antimicrobial agents are typically selected from the group consisting of the methyl and propyl esters of phydroxybenzoic acid (i.e., methyl and propylparaben), sodium benzoate, sorbic acid, imidurea, and combinations thereof.

Administration of a compound may be carried out using any appropriate mode of administration. Thus, administration can be, for example, oral, parenteral, topical, transdermal, transmucosal (including rectal and vaginal), sublingual, by inhalation, or via an implanted reservoir in a dosage form.

Depending on the intended mode of administration, the pharmaceutical formulation may be a solid, semi-solid or liquid, such as, for example, a tablet, a capsule, a caplet, a liquid, a suspension, an emulsion, a suppository, granules, pellets, beads, a powder, or the like, preferably in unit dosage form suitable for single administration of a precise dosage. Suitable pharmaceutical compositions and dosage forms may be prepared using conventional methods known to those in the field of pharmaceutical formulation and described in the pertinent texts and literature, e.g., in Remington: The Science and Practice of Pharmacy (Easton, Pa.: Mack Publishing Co., 1995).

Preferably, the pharmaceutical compositions for use or as used according to the present invention can be administered parenterally to a subject/patient in need of such treatment. The term "parenteral" as used herein is intended to include subcutaneous (dermal or subdermal), intravenous, and intramuscular injection or implantation (e.g., subcutaneously or intramuscularly or by intramuscular injection).

Preparations according to this invention for parenteral administration include sterile aqueous and nonaqueous solutions, suspensions, and emulsions. Injectable aqueous solutions contain the active agent in water-soluble form. Examples of nonaqueous solvents or vehicles include fatty oils, such as olive oil and corn oil, synthetic fatty acid esters, such as ethyl oleate or triglycerides, low molecular weight alcohols such as propylene glycol, synthetic hydrophilic polymers such as polyethylene glycol, liposomes, and the like. Parenteral formulations may also contain adjuvants such as solubilizers, preservatives, wetting agents, emulsifiers, dispersants, and stabilizers, and aqueous suspensions may contain substances that increase the viscosity of the suspension, such as sodium carboxymethyl cellulose, sorbitol, and dextran. Injectable formulations are rendered sterile by incorporation of a sterilizing agent, filtration through a bacteria-retaining filter, irradiation, or heat. They can also be manufactured using a sterile injectable medium. The active agent may also be in dried, e.g., lyophilized, form that may be rehydrated with a suitable vehicle immediately prior to administration via injection.

The quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals.

A carrier for parenteral administration can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin. It is also advantageous to include one or more cells or tissues which may supplement the use of the composition of the present invention. For example, it is preferred to include adipose tissue or cells, dermal fibroblasts or combination of thereof.

Suitable preservatives for use in solution include benzalkonium chloride, benzethonium chloride, chlorobutanol, thimerosal and the like. Suitable buffers include boric acid, sodium and potassium bicarbonate, sodium and potassium borates, sodium and potassium carbonate, sodium acetate, sodium biphosphate and the like, in amounts sufficient to maintain the pH at between about pH 6 and pH 8, and preferably, between about pH 7 and pH 7.5. Suitable tonicity agents are dextran 40, dextran 70, dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose and the like.

The compositions can be formulated for parenteral administration by dissolving, suspending or emulsifying in an aqueous or nonaqueous solvent. Vegetable (e.g., sesame oil, peanut oil) or similar oils, synthetic aliphatic acid glycerides, esters of higher aliphatic acids and propylene glycol are examples of nonaqueous solvents. Aqueous solutions such as Hank's solution, Ringer's solution or physiological saline buffer can also be used. In all cases the form must be sterile and must be fluid to the extent that easy syringability exists. It must be stable under the conditions of manufacture and storage and must be preserved against the contaminating action of microorganisms, such as bacteria and fungi.

Solutions of active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly, concentrated solutions for subcutaneous or intramuscular injection is also contemplated. In this regard, the use of DMSO as solvent is preferred as this will result in extremely rapid penetration, delivering high concentrations of the active compound(s) or agent(s) to a small area.

The present invention also provides kits for use in a therapeutic method for performing soft tissue augmentation and as used in a non-therapeutic method for performing soft tissue augmentation. Such kits can be prepared from readily available materials and reagents and can come in a variety of embodiments. For example, such kits can comprise, in an amount sufficient for at least one treatment, any one or more of the following materials: elastin and collagen isolated by methods, sterilized buffers (e.g., phosphate buffered salt) or water, other reagents necessary or helpful to perform the method, and instructions. Typically, instructions include a tangible expression describing reagent concentration or at least one method parameter, such as the amount of reagent to be used, maintenance time periods for reagents, and the like, to allow the user to carry out the methods described above. In a preferred embodiment of the invention, a kit comprises a means for delivery. Such means can include, by way of illustration and not limitation, a small syringe (22 to 30-gauge), a large syringe (13 to 19-gauge) and equipment used in endoscopic or percutaneous discectomy procedures. The reagents can be provided in solution, as suspensions, or as a substantially dry powder, e.g., in lyophilized form, either independently or in a mixture of components to improve ease of use. Where a degradable reagent is provided, conditions are chosen so as to stabilize the reagent, e.g., storage at lower temperature, addition of stabilizing agents (e.g., glycerol or a reducing agent). Unstable reagents can be provided together with or separately from the more stable components of the kit.

Further disclosed is the preparation of an elastin sheet for tissue repair or regeneration. Such a sheet may be generated by removing all or some other non-elastin components from intact native or engineered tissues, creating a porous elastin scaffold. A sheet can also be constructed by agglomeration of purified elastin particles. Either sheet type may be used in a hydrated form, or dried via lyophilization or another suitable drying method and then used in a dehydrated form. Dehydrated sheets can be rehydrated prior to implantation.

The present invention is further illustrated by the following examples that should not be construed as limiting in any way.

### EXAMPLES

### Example 1

Example 1 shows the isolation of elastin from native aorta. Elastin is also purified from aorta. The aorta can be obtained from any mammalian species, including but not limited to human, bovine, porcine, equine, or ovine. Alternatively, elastin can be isolated from other connective tissues that contain elastin, including skin, tendon, ligament, etc. Aorta is advantageous for isolation of elastin, since aorta is composed of approximately 30% elastin by dry weight. The process for isolating elastin involves a salt-based decellularization step, followed by boiling in 0.1 N NaOH and then extraction in hydrophobic solvents. Elastin isolation according to this example has unexpected properties when implanted in vivo, as shown in Example 2 (below). Steps for purifying elastin from aorta according to the present invention are as follows:
1. Obtain wet weight of aorta. Aorta is preferably fresh or non-frozen.
2. Shred aorta using a blender or some other device in distilled water.
3. Extract shredded tissues at 1-hour intervals in 0.9% NaCl solution at 4° C with shaking.
4. Repeat NaCl extraction until protein assay shows no soluble protein extraction.
5. Suspend samples in boiling 0.1 N NaOH solution, boil for 10-80 minutes.
6. Discard NaOH solution, then, rinse with distilled water.
7. Extract elastin 3 times, 30 minutes each, with 100% ethanol at room temperature.
8. Extract elastin in 50% ethanol/50% diethyl ether for 1 hour at room temperature.
9. Extract elastin in 100% diethyl ether for 1 hour at room temperature.
10. Decant ether, dry overnight. Obtain final weight.
11. Grind or pulverize to create injectable and insoluble elastin particles.

The amino acid analysis is performed, along with the HPLC analysis for desmosine cross-links, of elastin that is purified from aorta using the above method. For these experiments, a total of 6 different human aortas are treated using this protocol, and amino acid analysis is performed on 4 of the 6 samples. Desmosine quantification is performed on all samples as summarized in Table 1.

**TABLE 1: AA analysis and Desmosine for elastin from human aorta: (per 1.000 total residues)**

| **Amino Acid** | **Sample 54** | **Sample 55** | **Sample 56** | **Sample 57** | **Expected** |
|---|---|---|---|---|---|
| *cys | 0 | 0 | | | 3 |
| asx | 7 | 7 | 5 | 6 | 2 |
| thr | 9 | 8 | 5 | 5 | 14 |
| ser | 6 | 6 | 3 | 3 | 9 |
| glx | 21 | 21 | 20 | 19 | 3 |
| pro | 116 | 116 | 111 | 111 | 129 |
| gly | 332 | 331 | 353 | 353 | 312 |
| ala | 263 | 263 | 261 | 259 | 239 |
| val | 114 | 116 | 127 | 127 | 137 |
| *met | 0 | 0 | 0 | 0 | 0 |
| ile | 23 | 25 | 21 | 21 | 24 |
| leu | 60 | 61 | 55 | 58 | 65 |
| *tyr | 13 | 14 | 5 | 3 | 23 |
| phe | 24 | 25 | 19 | 21 | 24 |
| his | 0 | 0 | 0 | 0 | 0 |
| lys | 6 | 7 | 11 | 9 | 9 |
| arg | 5 | 4 | 5 | 5 | 9 |
| Des (pM/mg) | 12332 | 13291 | 19793 | 11904 | |

| | | | | | |
|---|---|---|---|---|---|
| * Cys, Tyr and Met are partially destroyed during acid hydrolysis Desmosine units are in (pico Moles/mg Protein) | | | | | |

As shown in Table 1, the values of alanine are well above 200 residues per 1,000 total residues, and values of valine are well above 70 residues per 1,000 residues. These are consistent with high purity elastin protein. In addition, values of Desmosine cross-links are very high, and are comparable to or higher than those reported for elastin preparations from a variety of species (see Table 2):

**Table 2: Desmosine from aortas of different species (pM/mg protein)**

| picomole/mg protein | |
|---|---|
| Cow | 12573 |
| Pig | 13934 |
| Monkey | 10948 |
| Rat | 6266 |
| Dog | 12942 |

Elastin that is isolated in accordance with the present invention has a fibrous structure and a small particle size. As shown in Figure 1, fibers are typically less than 200 microns in length, and can therefore easily pass through a syringe and fine-gauge needle for injection. Some fibers are longer than 200 microns. As is clear from Figure 1, fibers of elastin having dimensions on the order of microns have molecular weights that far exceed 100kDa, since the typical dimensions of molecules with MW=100 kDa are on the order of tens of nanometers.

Elastin that is isolated in accordance with the present invention can also be suspended in various carriers. One example is to suspend the elastin in a collagen gel, such as porcine collagen gel. As shown in Figure 2, porcine collagen gels that have 10-20 mg/mL of collagen (obtained from Medical Biomaterial Products GmbH, Germany) appear clear under phase-contrast microscopy (Figure 2A), but serve as good suspension media for elastin that is produced in accordance with the current invention (Figure 2B, fibrous particulates in collagen gel are elastin fibers).

### Example 2

Example 2 shows that purified elastin in vivo resists host cell infiltration. Implantation of substances into the subcutaneous space can elicit adverse host responses, including inflammation, recruitment of fibroblasts and fibrous encapsulation, recruitment of leukocytes and foreign body giant cells, calcification, scarring, and immune response with generation of antibodies or activated T-cell responses. Adverse host responses tend to be more severe for xenogeneic proteins that for allogeneic or for autologous proteins. To directly test the adverse host response to xenogeneic elastin, as compared to xenogeneic collagen, we implanted purified preparations of both human collagen and elastin into porcine subcutaneous tissue. The human elastin was isolated from aorta according to the present invention using salt-based decellularization followed by NaOH extraction as described in Example 1. After 4 months, xenogeneic human collagen (which is xenogeneic to porcine recipients) showed extensive host cell inflammatory response and infiltration on H&E staining (Figure 3A). In contrast, human elastin showed minimal host cell infiltration and no inflammatory cells, and no adverse response on H&E staining, which is in contrast to human collagen (Figure 3B). The presence of human elastin in the subcutaneous space is confirmed by Movats staining, which shows elastin is present and is densely black staining (Figure 3C). Hence, xenogeneic elastin that is prepared in accordance with the present invention does not induce adverse host response, as assessed by cellular infiltration and inflammation. This result is notable because purified xenogeneic collagen does induce significant adverse host response. In addition, Alizarin red staining for calcium deposition showed that no calcium was deposited in any sample of elastin at 4 months, showing excellent resistance of elastin to calcification.

### Example 3

Example 3 shows that purified elastin can be delivered in different carriers. To demonstrate that elastin that is prepared in accordance with the present invention could be injected subcutaneously using different carriers, we injected human elastin that was suspended into either human collagen or into crosslinked hyaluronic acid (Restylane) into porcine recipients. As shown in Figure 4, the elastin (which stains black) persists well subcutaneously for at least 4 months when suspended in either human collagen or in hyaluronic acid. In these images, subcutaneous fat stains white, dermis stains brown, and elastin stains black. It is notable that Figure 4 shows that human collagen carrier is largely absent at 4 months while human elastin persists, showing the superior persistence of elastin in this model. In addition, elastin is well dispersed in Restylane carrier, and shows superior persistence to Restylane in quantitative porcine implantation studies. Hence, elastin that is prepared in accordance with the present invention shows better persistence than several dermal fillers currently in use, including collagen and crosslinked hyaluronic acid.

### Example 4

Example 4 shows that purified elastin in vivo resists encapsulation and fibrosis. Elastin was injected subcutaneously into pigs and examined after 2 months. Elastin was injected in a collagen carrier and compared to crosslinked hyaluronic acid (Restylane). At two months in porcine recipients, Restylane samples exhibited frequent fibrous encapsulation (Figure 5B, arrow), which is an adverse host response to the injected hyaluronic acid. In contrast, no elastin samples elicited fibrous encapsulation (Figure 5A). This shows the resistance of the elastin of the invention to adverse host responses of fibrosis and encapsulation. In addition, Alizarin red staining for calcium deposition showed that no calcium was deposited in any sample of elastin at 2 months, showing excellent resistance of the elastin to calcification.

### Example 5

Example 5 shows that purified elastin may be xenogeneic or allogeneic to the recipient. Elastin was purified according to the present invention and suspended in porcine collagen carrier. Elastin was either human (at 45 mg/mL final concentration) or porcine (at 30 mg/mL final concentration) in porcine collagen (at 10 mg/mL concentration). Figure 6 shows host response after 1 month of implantation of porcine elastin in porcine collagen, showing good persistence of elastin and minimal host cellular response. Figure 7 shows host response after 1 month of implantation of human elastin in porcine collagen. Low power Movats (Fig. 7A) shows good persistence of elastin while higher power H&E stain (Fig. 7B) shows minimal host cell infiltration, even at 1 month time point which would generally be the time of highest host cell response and local inflammation. This example shows that elastin prepared according to the present invention induces minimal response in the recipient, regardless of whether the elastin is allogeneic or xenogeneic to the recipient. This example also shows that purified elastin can be successfully injected and is well tolerated by the host, regardless of whether the source of the elastin is either human or porcine.

## Claims

1. A composition for use in a therapeutic method for soft tissue augmentation in a human subject, wherein said composition comprises isolated non-human elastin and a pharmaceutically acceptable carrier, wherein the elastin has a molecular weight greater than 100 kDa and is substantially insoluble in water, and wherein the non-human elastin is isolated from non-frozen vascular tissue.

2. Use of a composition in a non-therapeutic method for soft tissue augmentation in a human subject, wherein said composition comprises isolated non-human elastin and a pharmaceutically acceptable carrier, wherein the elastin has a molecular weight greater than 100 kDa and is substantially insoluble in water, and wherein non-human the elastin is isolated from non-frozen vascular tissue.

3. The composition for the use of claim 1 further comprising isolated collagen, optionally wherein the isolated collagen is non-human collagen, or wherein the isolated collagen is human collagen.

4. The use of the composition of claim 2, wherein the composition further comprises isolated non-human collagen.

5. The composition for the use of claim 1 or the use of the composition of claim 2, wherein the composition further comprises glycosaminoglycans, or
wherein the composition further comprises adipose tissue, or
wherein the composition further comprises dermal fibroblasts, or
wherein the composition further comprises one or more active agents selected from the group consisting of one or more anti-inflammatory agents, tissue formation agents, adipose tissue formation agents, anesthetics, antioxidants, heparin, epidermal growth factor, transforming growth factor, transforming growth factor-β, platelet-derived growth factor, fibroblast growth factor, connective tissue activating peptides, β-thromboglobulin, insulin-like growth factors, tumor necrosis factors, interleukins, colony stimulating factors, erythropoietin, nerve growth factors, interferons or combinations thereof.

6. The composition for the use of claim 1 or the use of the composition of claim 2, wherein the isolated elastin is
(a) cross-linked; or
(b) present in an amount of from about 2 to about 100 mg/ml of isolated elastin.

7. The composition for the use of claim 1 or the use of the composition of claim 2, wherein said composition does not induce calcification, fibrosis or encapsulation *in vivo* upon administration to a subject.

8. A dermal or subdermal filler comprising the composition for the use of claim 1.

9. The use of the composition of claim 2 wherein the composition is contained within a dermal or subdermal filler.

10. A kit for augmentation of a soft tissue comprising the composition for the use of claim 1, a syringe, a sterile wrapper surrounding said syringe and one or more reagents.

11. Use of a kit in a non-therapeutic method for soft tissue augmentation in a human subject, wherein said kit comprises a composition according to claim 2, a syringe, a sterile wrapper surrounding said syringe and one or more reagents.

12. The kit for the use of claim 10 or the use of the kit of claim 11, wherein said reagents are selected from the group consisting of heparin, epidermal growth factor, transforming growth factor-alpha, transforming growth factor-beta, platelet-derived growth factor, fibroblast growth factor, connective tissue activating peptides, β-thromboglobulin, insulin-like growth factors, tumor necrosis factors, interleukins, colony stimulating factors, erythropoietin, nerve growth factors, interferons, osteogenic factors and bone morphogenic proteins.

13. The composition for use of claim 1, wherein the soft tissue augmentation improves a therapeutic condition selected from the group consisting of cleft lips, correction of minor deformities due to disease, deformities of the vocal cords or glottis, deformities of the lip, breast deformities, chin deformities, cheek and/or nose deformities, acne, surgical scars and scars due to radiation damage or trauma scars.

14. The use of the composition of claim 2, wherein the soft tissue augmentation improves a non-therapeutic condition selected from the group consisting of lines, folds, wrinkles, minor facial depressions, correction of minor deformities due to aging, crow's feet and the orbital groove around the eye, augmentation and rhytids.

## Patentansprüche

1. Zusammensetzung zur Verwendung in einem therapeutischen Verfahren zur Weichgewebeaugmentation bei einem menschlichen Probanden, wobei die Zusammensetzung isoliertes nichtmenschliches Elastin und einen pharmazeutisch akzeptablen Trägerstoff umfasst, wobei das Elastin ein Molekulargewicht von mehr als 100 kDa aufweist und in Wasser im Wesentlichen unlöslich ist und wobei das nichtmenschliche Elastin aus nicht gefrorenem Gefäßgewebe isoliert ist.

2. Verwendung einer Zusammensetzung in einem nichttherapeutischen Verfahren zur Weichgewebeaugmentation bei einem menschlichen Probanden, wobei die Zusammensetzung isoliertes nichtmenschliches Elastin und einen pharmazeutisch akzeptablen Trägerstoff umfasst, wobei das Elastin ein Molekulargewicht von mehr als 100 kDa aufweist und in Wasser im Wesentlichen unlöslich ist und wobei das nichtmenschliche Elastin aus nicht gefrorenem Gefäßgewebe isoliert ist.

3. Zusammensetzung zur Verwendung nach Anspruch 1, die weiterhin isoliertes Kollagen umfasst, gegebenenfalls wobei das isolierte Kollagen nichtmenschliches Kollagen ist oder wobei das isolierte Kollagen menschliches Kollagen ist.

4. Verwendung der Zusammensetzung nach Anspruch 2, wobei die Zusammensetzung weiterhin isoliertes nichtmenschliches Kollagen umfasst.

5. Zusammensetzung zur Verwendung nach Anspruch 1 oder Verwendung der Zusammensetzung nach Anspruch 2, wobei die Zusammensetzung weiterhin Glykosaminoglykane umfasst oder
wobei die Zusammensetzung weiterhin Fettgewebe umfasst oder
wobei die Zusammensetzung weiterhin Hautfibroblasten umfasst oder
wobei die Zusammensetzung weiterhin einen oder mehrere Wirkstoffe umfasst, die aus der Gruppe bestehend aus einem oder mehreren Entzündungshemmern, Gewebebildungsmitteln, Fettgewebebildungsmitteln, Anästhetika, Antioxidationsmitteln, Heparin, epidermalem Wachstumsfaktor, transformierendem Wachstumsfaktor, transformierendem Wachstumsfaktor-β, Thrombozytenwachstumsfaktor, Fibroblastenwachstumsfaktor, Bindegewebe aktivierenden Peptiden, β-Thromboglobulin, insulinähnlichen Wachstumsfaktoren, Tumornekrosefaktoren, Interleukinen, koloniestimulierenden Faktoren, Erythropoetin, Nervenwachstumsfaktoren, Interferonen oder Kombinationen davon ausgewählt sind.

6. Zusammensetzung zur Verwendung nach Anspruch 1 oder Verwendung der Zusammensetzung nach Anspruch 2, wobei das isolierte Elastin
(a) vernetzt ist oder
(b) in einer Menge von etwa 2 bis etwa 100 mg/ml isoliertes Elastin vorliegt.

7. Zusammensetzung zur Verwendung nach Anspruch 1 oder Verwendung der Zusammensetzung nach Anspruch 2, wobei die Zusammensetzung bei Verabreichung an einen Probanden keine Kalzifikation, Fibrose oder Einkapselung *in vivo* induziert.

8. Dermaler oder subdermaler Füller, der die Zusammensetzung zur Verwendung nach Anspruch 1 umfasst.

9. Verwendung der Zusammensetzung nach Anspruch 2, wobei die Zusammensetzung in einem dermalen oder subdermalen Füller enthalten ist.

10. Kit zur Augmentation eines Weichgewebes, das die Zusammensetzung zur Verwendung nach Anspruch 1, eine Spritze, eine sterile Hülle, die die Spritze umschließt, und ein oder mehrere Reagenzien umfasst.

11. Verwendung eines Kits in einem nichttherapeutischen Verfahren zur Weichgewebeaugmentation bei einem menschlichen Probanden, wobei das Kit eine Zusammensetzung nach Anspruch 2, eine Spritze, eine sterile Hülle, die die Spritze umschließt, und ein oder mehrere Reagenzien umfasst.

12. Kit zur Verwendung nach Anspruch 10 oder Verwendung des Kits nach Anspruch 11, wobei die Reagenzien aus der Gruppe bestehend aus Heparin, epidermalem Wachstumsfaktor, transformierendem Wachstumsfaktor-alpha, transformierendem Wachstumsfaktor-beta, Thrombozytenwachstumsfaktor, Fibroblastenwachstumsfaktor, Bindegewebe aktivierenden Peptiden, β-Thromboglobulin, insulinähnlichen Wachstumsfaktoren, Tumornekrosefaktoren, Interleukinen, koloniestimulierenden Faktoren, Erythropoetin, Nervenwachstumsfaktoren, Interferonen, osteogenetischen Faktoren oder knochenmorphogenetischen Proteinen ausgewählt sind.

13. Zusammensetzung zur Verwendung nach Anspruch 1, wobei die Weichgewebeaugmentation einen therapeutischen Zustand verbessert, der aus der Gruppe bestehend aus Lippenspalten, einer Korrektur von weniger ausgeprägten Deformitäten aufgrund einer Krankheit, Deformitäten der Stimmbänder oder Glottis, Deformitäten der Lippe, Brustdeformitäten, Kinndeformitäten, Wangen- und/oder Nasendeformitäten, Akne, chirurgischen Narben und Narben aufgrund von Strahlenschädigung oder Traumanarben ausgewählt ist.

14. Verwendung der Zusammensetzung nach Anspruch 2, wobei die Weichgewebeaugmentation einen nichttherapeutischen Zustand verbessert, der aus der Gruppe bestehend aus Linien, Falten, Runzeln, weniger ausgeprägten Gesichtsgrübchen, einer Korrektur von weniger ausgeprägten Deformitäten aufgrund von Alterung, Augenfältchen und der Höhle um das Auge, einer Augmentation und Rhytiden ausgewählt ist.

## Revendications

1. Composition destinée à être utilisée dans une procédure thérapeutique d'augmentation du volume de tissus mous chez un sujet humain, où ladite composition comprend une élastine non humaine isolée et un véhicule pharmaceutiquement acceptable, où l'élastine a une masse moléculaire supérieure à 100 kDa et est essentiellement insoluble dans l'eau et où l'élastine non humaine est isolée d'un tissu vasculaire non congelé.

2. Utilisation d'une composition dans une procédure non thérapeutique d'augmentation du volume de tissus mous chez un sujet humain, où ladite composition comprend une élastine non humaine isolée et un véhicule pharmaceutiquement acceptable, où l'élastine a une masse moléculaire supérieure à 100 kDa et est essentiellement insoluble dans l'eau et où l'élastine non humaine est isolée d'un tissu vasculaire non congelé.

3. Composition pour l'utilisation selon la revendication 1 qui comprend en outre un collagène isolé, en option où le collagène isolé est un collagène non humain ou où le collagène isolé est un collagène humain.

4. Utilisation de la composition selon la revendication 2, où la composition comprend en outre un collagène non humain isolé.

5. Composition pour l'utilisation selon la revendication 1 ou utilisation de la composition selon la revendication 2, où la composition comprend en outre des glycosaminoglycanes ou
où la composition comprend en outre un tissu adipeux ou
où la composition comprend en outre des fibroblastes dermiques ou
où la composition comprend en outre un ou plusieurs agents actifs sélectionnés dans le groupe consistant en un ou plusieurs des suivants : agents anti-inflammatoires, agents agissant sur la formation de tissus, agents agissant sur la formation de tissus adipeux, anesthésiques, antioxydants, héparine, facteur de croissance épidermique, facteur de croissance transformant, facteur de croissance transformant β, facteur de croissance dérivé des plaquettes, facteur de croissance fibroblastique, peptides activateurs du tissu conjonctif, β-thromboglobuline, facteurs de croissance analogues à l'insuline, facteurs de nécrose tumorale, interleukines, facteurs de stimulation de colonies, érythropoïétine, facteurs de croissance nerveuse, interférons ou combinaisons de ceux-ci.

6. Composition pour l'utilisation selon la revendication 1 ou utilisation de la composition selon la revendication 2, où l'élastine isolée est
(a) réticulée ; ou
(b) présente à une quantité qui va d'environ 2 à environ 100 mg/ml d'élastine isolée.

7. Composition pour l'utilisation selon la revendication 1 ou utilisation de la composition selon la revendication 2, où ladite composition n'induit pas une calcification, une fibrose ou une encapsulation *in vivo* lors de l'administration à un sujet.

8. Produit de comblement dermique ou sous-dermique comprenant la composition pour l'utilisation selon la revendication 1.

9. Utilisation de la composition selon la revendication 2, où la composition est contenue dans un produit de comblement dermique ou sous-dermique.

10. Nécessaire permettant d'augmenter le volume d'un tissu mou qui comprend la composition pour l'utilisation selon la revendication 1, une seringue, un conditionnement stérile entourant ladite seringue et un ou plusieurs réactifs.

11. Utilisation d'un nécessaire dans une procédure non thérapeutique d'augmentation du volume de tissus mous chez un sujet humain, où ledit nécessaire comprend une composition selon la revendication 2, une seringue, un conditionnement stérile entourant ladite seringue et un ou plusieurs réactifs.

12. Nécessaire pour l'utilisation selon la revendication 10 ou utilisation du nécessaire selon la revendication 11, où lesdits réactifs sont sélectionnés dans le groupe consistant en les suivants : héparine, facteur de croissance épidermique, facteur de croissance transformant alpha, facteur de croissance transformant bêta, facteur de croissance dérivé des plaquettes, facteur de croissance fibroblastique, peptides activateurs du tissu conjonctif, β-thromboglobuline, facteurs de croissance analogues à l'insuline, facteurs de nécrose tumorale, interleukines, facteurs de stimulation de colonies, érythropoïétine, facteurs de croissance nerveuse, interférons, facteurs ostéogéniques et protéines morphogéniques osseuses.

13. Composition pour l'utilisation selon la revendication 1, où l'augmentation du volume de tissus mous produit une amélioration à visée thérapeutique dans une situation sélectionnée dans le groupe consistant en les suivantes :
fissure labiale, correction de difformités mineures d'origine pathologique, difformités des cordes vocales ou de la glotte, difformités des lèvres, difformités des seins, difformités du menton, difformités des joues et/ou du nez, acné, cicatrices chirurgicales et cicatrices secondaires à des lésions post-radiques ou à un traumatisme.

14. Utilisation de la composition selon la revendication 2, où l'augmentation du volume de tissus mous produit une amélioration à visée non thérapeutique dans une situation sélectionnée dans le groupe consistant en les suivantes :
lignes, plis, rides, dépressions faciales mineures,
correction de difformités mineures dues au vieillissement,
pattes d'oie et sillons jugo-palpébraux, augmentation de volume et rhytides.
